(19) Europäisches Patentamt
European Patent Office
Office européen des brevets

(11) **EP 4 056 166 A1**

(12) **EUROPEAN PATENT APPLICATION**

(43) Date of publication:
**14.09.2022 Bulletin 2022/37**

(21) Application number: **22165661.4**

(22) Date of filing: **13.03.2020**

(51) International Patent Classification (IPC):
**A61K 8/41** (2006.01)     **A61K 8/46** (2006.01)
**A61K 8/49** (2006.01)     **A61Q 17/04** (2006.01)

(52) Cooperative Patent Classification (CPC):
**A61Q 17/04; A61K 8/415; A61K 8/466; A61K 8/4946**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **15.03.2019 EP 19163166**

(62) Document number(s) of the earlier application(s) in accordance with Art. 76 EPC:
**20710522.2 / 3 937 897**

(71) Applicant: **BASF SE**
**67056 Ludwigshafen am Rhein (DE)**

(72) Inventors:
• **SOHN, Myriam**
**79639 Grenzach-Wyhlen (DE)**

• **KRUS, Stanislaw**
**79639 Grenzach-Wyhlen (DE)**
• **SCHNYDER, Marcel**
**79639 Grenzach-Wyhlen (DE)**
• **ACKER, Stephanie**
**79639 Grenzach-Wyhlen (DE)**

(74) Representative: **Maiwald Patent- und Rechtsanwaltsgesellschaft mbH**
**Elisenhof**
**Elisenstraße 3**
**80335 München (DE)**

Remarks:
This application was filed on 30-03-2022 as a divisional application to the application mentioned under INID code 62.

(54) **EFFICIENT SUNSCREEN COMPOSITIONS WITH DIETHYLAMINO HYDROXYBENZOYL HEXYL BENZOATE AND WATER SOLUBLE UVA FILTER**

(57) The present invention relates to sunscreen or daily care compositions comprising hexyl 2-[4-(diethylamino)-2-hydroxybenzoyl]benzoate (INCI diethylamino hydroxybenzoyl hexyl benzoate) and at least one water soluble UVA filter, wherein the composition does not comprise 2-hydroxy-4-methoxybenzophenone (INCI oxybenzone) and ethylhexyl-2-cyano-3,3-diphenyl-acrylate (INCI octocrylene). Furthermore, the present invention relates to the use of said composition to reduce fabric staining and to facilitate the washability of a sunscreen or daily care composition.

Processed by Luminess, 75001 PARIS (FR)

**Description**

The present invention relates to sunscreen or daily care compositions comprising hexyl 2-[4-(diethylamino)-2-hydroxybenzoyl]benzoate (INCI diethylamino hydroxybenzoyl hexyl benzoate) and at least one water soluble UVA filter, wherein the composition does not comprise 2-hydroxy-4-methoxybenzophenone (INCI oxybenzone) and ethylhexyl-2-cyano-3,3-diphenyl-acrylate (INCI octocrylene). Furthermore, the present invention relates to the use of said composition to reduce fabric staining and to facilitate the washability of a sunscreen or daily care composition.

UV radiation causes harmful effects on the human skin. Beside the acute effect of sunburn of the skin, UV radiation is also known to increase the risk of skin cancer. Furthermore, long time exposure to UV-A and UV-B light can cause phototoxic and photo allergenic reactions on the skin and can accelerate skin aging.

To protect the human skin from UV radiation, various sun protecting UV filters (also referred to as UV absorbers) exist including UV-A filter, UV-B filter and broadband filters. These filters are added to sunscreen or cosmetic compositions. The UV filters are either organic or inorganic, particulate or non-particulate compounds, of which all have a high absorption efficacy in the UV-light range. In general, UV light can be divided into UV-A radiation and UV-B radiation. Depending on the position of the absorption maxima, UV-filters are divided into UV-A and UV-B filters. In case an UV-filter absorbs both, UV-A and UV-B light, it is referred to as a broadband absorber.

Since 2006, the EU commission has recommended that all sunscreen or cosmetic compositions should have an UV-A protection factor, which is at least one third of the labelled sun protection factor (SPF), wherein the sun protection factor refers mainly to the UV-B protection.

However, the UV filters known in the prior art which are used in sunscreen or cosmetic compositions have certain disadvantages. In particular, it is referred to the disadvantage of certain UV filter, which are frequently under discussion due to their health and environmental concern, although they are approved for being used in sunscreen or cosmetic compositions. UV filter under discussion are for example octocrylene, oxybenzone, homosalate, ethylhexyl salicylate or ethylhexyl methoxy cinnamate. Therefore, there is a need for sunscreen or cosmetic compositions of the daily use, which are efficient for sun protection but free of UV filter under discussion.

Furthermore, sunscreen or cosmetic compositions can adhere on fabrics or textiles and cause fabric staining. In view of the low solubility of certain UV filters in water, the resulting stains are hard to remove during washing of the fabrics.

Accordingly, it is desired to reduce fabric staining and/or facilitate the washability of sunscreen compositions from textiles without reducing the UV-A protection.

EP 2 837 407 A2 discloses odor stable, octocrylene free cosmetic compositions comprising one or more UV filter selected from triazine derivatives, titanium dioxide, butyl methoxydibenzoylmethane and / or diethylamino hydroxybenzoyl hexyl benzoate, wherein the composition is free of ethylhexyl salicylate, octocrylene, homomenthylsalicylate and ethylhexyl methoxy cinnamate.

EP 3 093 008 A1 refers to a sunscreen composition which is free of octocrylene and comprises diethylamino hydroxybenzoyl hexyl benzoate, phenylbenzimidazole sulfonic acid, and one or more salicylates selected from ethylhexyl salicylate and homosalate.

EP 3 093 007 A1 discloses a cosmetic composition comprising a UV filter combination of diethylamino hydroxybenzoyl hexyl benzoate, ethylhexyl triazone and one or more salicylates selected from ethylhexyl salicylate and homosalate, wherein the composition may further be free of oxybenzone, octocrylene and 4-methylbenzylidene camphor.

EP 3 093 006 relates to an alcohol containing, octocrylene free sunscreen composition. The cosmetic composition comprises a UV filter combination of diethylamino hydroxybenzoyl hexyl benzoate, ethylhexyl triazone, and bis-ethylhexylphenol methoxyphenyl triazine, wherein the composition comprises one or more alcohols.

EP 3 195 853 A1 discloses an octocrylene free sunscreen composition, wherein the cosmetic composition comprises a UV filter combination of diethylamino hydroxybenzoyl hexyl benzoate, diethyl butamido triazone and ethylhexyl triazone.

EP 3 351 236 A1 relates to a cosmetic composition comprising a UV filter combination of diethylamino hydroxybenzoyl hexyl benzoate, butyl methoxydibenzoylmethane and polyglycerol fatty acid esters, wherein the composition is free of 4-methylbenzylidene camphor, oxybenzone, ethylhexyl methoxycinnamate, isoamyl p-methoxycinnamate and octocrylene.

US 2003/0161793 A1 discloses a topically applicable cosmetic/dermatological sunscreen composition devoid of any p-methylbenzylidene camphor but contains at least one UV-screening dibenzoylmethane compound and at least one UV-screening amino-substituted 2-hydroxybenzophenone compound of the structural formula (I).

DE 600 22 411 T2 discloses a cosmetic composition for topical application comprising at least one specific bis-resorcinyltriazine derivative and at least one UV filter comprising at least two benzoazolylgroups per molecule.

EP 3 354 253 A2 describes a process for the reduction of fabric staining by incorporating diethylamino hydroxybenzoyl hexyl benzoate in cosmetic compositions.

Therefore, it has been an object of the present invention to provide efficient sunscreen or daily care compositions. It has been another object of the present invention to provide efficient sunscreen or daily care compositions, which perfectly meet the consumer's demands. In this connection, it has been another object of the present invention to provide sunscreen or daily care compositions, which are free of UV filters under discussion. It has been another object of the present invention to provide compositions, which are suitable for use in sunscreen or daily care compositions in order to reduce fabric staining and facilitate the washability of textiles.

It has surprisingly been found that at least one of these objects can be achieved by the sunscreen or daily care composition according to the present invention.

In particular, the inventors of the present application found that the sunscreen or daily care composition according to the present invention provides an efficient UV-A and UV-B protection, nevertheless the composition is free of certain UV filter under discussion, i.e. octocrylene. Furthermore, it has surprisingly been found by the inventors of the present application that the sunscreen or daily care composition according to the present invention can be used to reduce fabric staining and facilitate the washability of the sunscreen or daily care composition from textiles.

Thus, according to one embodiment, the present invention relates to a sunscreen or daily care composition comprising

(i) hexyl 2-[4-(diethylamino)-2-hydroxybenzoyl]benzoate (INCI diethylamino hydroxybenzoyl hexyl benzoate); and
(ii) at least one water soluble UVA filter;

wherein the composition does not comprise 2-hydroxy-4-methoxybenzophenone (INCI oxybenzone) and ethylhexyl-2-cyano-3,3-diphenyl-acrylate (INCI octocrylene).

In a preferred embodiment of said composition, the sunscreen or daily care composition is free of parabens.

In another preferred embodiment of said composition, in the sunscreen or daily care composition the at least one water soluble UVA filter is selected from the group consisting of [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1 - bicyclo[2.2.1]heptanyl]methanesulfonic acid (INCI terephthalylidene dicamphor sulfonic acid), disodium phenyl dibenzimidazole tetrasulfonate, and the combination thereof.

In another preferred embodiment of said composition, the sunscreen or daily care composition comprises at least one further UVA filter selected from the group consisting of triazine derivatives, dibenzoylmethane derivatives, benzotriazole derivatives and combinations thereof.

In another preferred embodiment of said composition, in the sunscreen or daily care composition the at least one further UVA filter is selected from the group consisting of 4-(tert.-butyl)-4'-methoxydibenzoylmethane (INCI butyl methoxydibenzoylmethane), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane), 2-(2H-benzotriazol-2-yl)-6-(2-ethylhexyloxymethyl)-4-methyl-phenol and combinations thereof.

In another preferred embodiment of said composition, the sunscreen or daily care composition further comprises at least one UVB filter selected from the group consisting of 4,4',4"-(1,3,5-triazin-2,4,6-triyltriimino)tris-benzoic acid-tris(2-ethylhexyl)ester (INCI ethylhexyl triazone), malonate derivatives such as dimethicone diethyl benzalmalonate, diethylhexyl butamido triazone, and combinations thereof.

In another preferred embodiment of said composition, the sunscreen or daily care composition does not comprise 2-ethylhexyl-(2E)-3-(4-methoxyphenyl)acrylate (INCI ethylhexyl methoxy cinnamate).

In another preferred embodiment of said composition, the sunscreen or daily care composition is free of phenoxyethanol.

In another preferred embodiment of said composition, the sunscreen or daily care composition comprises hexyl 2-[4-(diethylamino)-2-hydroxybenzoyl]benzoate (INCI diethylamino hydroxybenzoyl hexyl benzoate) in an amount of from 1% to 10% by weight, based on the total weight of the composition.

In another preferred embodiment of said composition, the sunscreen or daily care composition comprises the at least one water soluble UVA filter in an amount of from 0,1% to 10% by weight, preferably in an amount of from 0.3% to 5% by weight, more preferably in an amount of from 0.5% to 3% by weight, based on the total weight of the composition.

In a more preferred embodiment of said composition, the sunscreen or daily care composition comprises

(i) hexyl 2-[4-(diethylamino)-2-hydroxybenzoyl]benzoate (INCI diethylamino hydroxybenzoyl hexyl benzoate); and
(ii) at least one water soluble UVA filter, which is [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1 -bicyclo[2.2.1]heptanyl]methanesulfonic acid (INCI terephthalylidene dicamphor sulfonic acid) and/or disodium phenyl dibenzimidazole tetrasulfonate;

wherein the composition does not comprise 2-hydroxy-4-methoxybenzophenone (INCI oxybenzone) and ethylhexyl-2-cyano-3,3-diphenyl-acrylate (INCI octocrylene); and wherein the composition is free of parabens.

In another preferred embodiment of said composition, the sunscreen or daily care composition is free of (RS)-

3

2-ethylhexyl-2-hydroxybenzoate (INCI ethylhexyl salicylate) and 3,3,5-trimethyl-cyclohexyl salicylate (homosalate).

In a second embodiment, the present invention relates to a sunscreen or daily care composition comprising

(i) hexyl 2-[4-(diethylamino)-2-hydroxybenzoyl]benzoate (INCI diethylamino hydroxybenzoyl hexyl benzoate); and
(ii) at least one water soluble UVA filter selected from disodium phenyl dibenzimidazole tetrasulfonate in an amount of from 1% to 10% by weight based on the total weight of the composition;

wherein the composition does not comprise 2-hydroxy-4-methoxybenzophenone (INCI oxybenzone), ethylhexyl-2-cyano-3,3-diphenyl-acrylate (INCI octocrylene) and 2-ethylhexyl-(2E)-3-(4-methoxyphenyl)acrylate (INCI ethylhexyl methoxy cinnamate).

In a preferred embodiment of said composition of said second embodiment, the sunscreen or daily care composition is free of parabens.

In another preferred embodiment of said composition of said second embodiment, the composition comprises at least one further UVA filter selected from the group consisting of triazine derivatives, dibenzoylmethane derivatives , benzotriazole derivatives and combinations thereof.

In another preferred embodiment of said composition of said second embodiment, the at least one further UVA filter is selected from the group consisting of 4-(tert.-butyl)-4'-methoxydibenzoylmethane (INCI butyl methoxydibenzoylmethane), 2,4-bis-{[4-(2-ethyl-hexyloxy)-2-hydroxy]-phenyl}-6-(4-methoxyphenyl)-1,3,5 triazine (INCI bis-ethylhexyloxy-phenol methoxyphenyl triazine), 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane), 2-(2H-benzotriazol-2-yl)-6-(2-ethylhexyloxymethyl)-4-methyl-phenol and combinations thereof.

In another preferred embodiment of said composition of said second embodiment, the composition further comprises at least one UVB filter selected from the group consisting of 4,4',4"-(1,3,5-triazin-2,4,6-triyltriimino)tris-benzoic acid-tris(2-ethylhexyl)ester (INCI ethylhexyl triazone), malonate derivatives such as dimethicone diethyl benzalmalonate, diethylhexyl butamido triazone, and combinations thereof.

In another preferred embodiment of said composition of said second embodiment, the composition is free of phenoxyethanol.

In another preferred embodiment of said composition of said second embodiment, the composition comprises hexyl 2-[4-(diethylamino)-2-hydroxybenzoyl]benzoate (INCI diethylamino hydroxybenzoyl hexyl benzoate) in an amount of from 1 % to 10 % by weight, based on the total weight of the composition.

In another preferred embodiment of said composition of said second embodiment, the composition comprises the at least one water soluble UVA filter in an amount of from 1 % to 8% by weight, preferably in an amount of from 1 % to 7% by weight, more preferably in an amount of from 1% to 6% by weight, based on the total weight of the composition.

In another preferred embodiment of said composition of said second embodiment, the composition is free of (RS)-2-ethylhexyl-2-hydroxybenzoate (INCI ethylhexyl salicylate) and 3,3,5-trimethyl-cyclohexyl salicylate (homosalate).

In another preferred embodiment of said composition of said second embodiment, the composition comprises at least one perfume.

In another aspect the present invention relates to the use of a sunscreen or daily care composition to reduce fabric staining, wherein the composition comprises

(i) hexyl 2-[4-(diethylamino)-2-hydroxybenzoyl]benzoate (INCI diethylamino hydroxybenzoyl hexyl benzoate); and
(ii) at least one water soluble UVA filter; and

wherein the composition does not comprise 2-hydroxy-4-methoxybenzophenone (INCI oxybenzone) and ethylhexyl-2-cyano-3,3-diphenyl-acrylate (INCI octocrylene).

In a third aspect the present invention relates to the use of a sunscreen or daily care composition to facilitate the washability from textiles, wherein the composition comprises

(i) hexyl 2-[4-(diethylamino)-2-hydroxybenzoyl]benzoate (INCI diethylamino hydroxybenzoyl hexyl benzoate); and
(ii) at least one water soluble UVA filter; and

wherein the composition does not comprise 2-hydroxy-4-methoxybenzophenone (INCI oxybenzone) and ethylhexyl-2-cyano-3,3-diphenyl-acrylate (INCI octocrylene).

In a preferred embodiment of said uses, the sunscreen or daily care composition is free of parabens.

In another preferred embodiment of said uses, in the sunscreen or daily care composition the at least one water soluble UVA filter is selected from the group consisting of [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1-bicyclo[2.2.1]heptanyl]methanesulfonic acid (INCI terephthalylidene dicamphor sulfonic acid), disodium phenyl dibenzimidazole tetrasulfonate, and the combination thereof.

In another preferred embodiment of said uses, the sunscreen or daily care composition comprises at least one further UVA filter selected from the group consisting of triazine derivatives, dibenzoylmethane derivatives , benzotriazole derivatives and combinations thereof.

In a more preferred embodiment of said uses, in the sunscreen or daily care composition the at least one further UVA filter is selected from the group consisting of 4-(tert.-butyl)-4'-methoxydibenzoylmethane (INCI butyl methoxydibenzoylmethane), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane), 2-(2H-benzotriazol-2-yl)-6-(2-ethylhexyloxymethyl)-4-methyl-phenol and combinations thereof.

In another preferred embodiment of said uses, the sunscreen or daily care composition further comprises at least one UVB filter selected from the group consisting of 4,4',4''-(1,3,5-triazin-2,4,6-triyltriimino)tris-benzoic acid-tris(2-ethylhexyl)ester (INCI ethylhexyl triazone), malonate derivatives such as dimethicone diethyl benzalmalonate, diethylhexyl butamido triazone, and combinations thereof.

In another preferred embodiment of said uses, the sunscreen or daily care composition does not comprise 2-ethylhexyl-(2E)-3-(4-methoxyphenyl)acrylate (INCI ethylhexyl methoxy cinnamate).

In another preferred embodiment of said uses, the sunscreen or daily care composition is free of phenoxyethanol.

In another preferred embodiment of said uses, the sunscreen or daily care composition comprises hexyl 2-[4-(diethylamino)-2-hydroxybenzoyl]benzoate (INCI diethylamino hydroxybenzoyl hexyl benzoate) in an amount of from 1% to 10% by weight, based on the total weight of the composition.

In another preferred embodiment of said uses, the sunscreen or daily care composition comprises the at least one water soluble UVA filter in an amount of from 0,1% to 10% by weight, preferably in an amount of from 0.3% to 5% by weight, even more preferably in an amount of from 0.5% to 3% by weight, based on the total weight of the composition.

In a more preferred embodiment of said uses, the sunscreen or daily care composition comprises

(i) hexyl 2-[4-(diethylamino)-2-hydroxybenzoyl]benzoate (INCI diethylamino hydroxybenzoyl hexyl benzoate); and
(ii) at least one water soluble UVA filter, which is [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1 -bicyclo[2.2.1]heptanyl]methanesulfonic acid (INCI terephthalylidene dicamphor sulfonic acid) and/or disodium phenyl dibenzimidazole tetrasulfonate;

wherein the composition does not comprise 2-hydroxy-4-methoxybenzophenone (INCI oxybenzone) and ethylhexyl-2-cyano-3,3-diphenyl-acrylate (INCI octocrylene); and wherein the composition is free of parabens.

In another preferred embodiment of said uses, the sunscreen or daily care composition is free of (RS)-2-ethylhexyl-2-hydroxybenzoate (INCI ethylhexyl salicylate) and 3,3,5-trimethyl-cyclohexyl salicylate (homosalate).

Before describing preferred embodiments of the present invention in detail, definitions important for understanding the present invention are given.

As used in this specification and in the appended claims, the singular forms of "a" and "an" also include the respective plurals unless the context clearly dictates otherwise. In the context of the present invention, the terms "about" and "approximately" denote an interval of accuracy that a person skilled in the art will understand to still ensure the technical effect of the feature in question. The term typically indicates a deviation from the indicated numerical value of $\pm 20\%$, preferably $\pm 15\%$, more preferably $\pm 10\%$, and even more preferably $\pm 5$ %. It is to be understood that the term "comprising" is not limiting. For the purposes of the present invention, the term "consisting of" is considered to be a preferred embodiment of the term "comprising of". If hereinafter a group is defined to comprise at least a certain number of embodiments, this is meant to also encompass a group, which preferably consists of these embodiments only.

Furthermore, the terms "first", "second", "third" or "(i)", "(ii)", "(iii)", "(iv)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

As used herein the term "free of" in the context that the composition of the present invention is free of a specific compound or group of compounds, which may be combined under a collective term, means that the composition does not comprise said compound or group of compounds in an amount of more than 0.8% by weight, based on the total weight of the composition. Furthermore, it is preferred that the composition according to the present invention does not comprise said compounds or group of compounds in an amount of more than 0.5% by weight, preferably the composition does not comprise said compounds or group of compounds at all. The same definition is applied for the term "does not comprise".

The term "sunscreen composition" refers to any topical product, which reflects and/or absorbs certain parts of UV radiation. Thus, the term "sunscreen composition" is to be understood as not only including sunscreen compositions, but also any cosmetic compositions that provide UV protection. The term "topical product" refers to a product that is applied to the skin and can refer, e.g., to sprays, lotions, creams, oils, or gels. The sunscreen composition may comprise one or more active agents, e.g., organic or inorganic UV filters, as well as other ingredients or additives, e.g., emulsifiers, emollients, viscosity regulators, stabilizers, preservatives, or fragrances.

The term "daily care composition" refers to any topical product, which reflects or absorbs certain parts of UV radiation and is used as an everyday care product for the human body, e.g., for face, body or hair. The daily care composition may comprise one or more active agents, e.g., organic or inorganic UV filters, as well as other ingredients or additives, e.g., emulsifiers, emollients, viscosity regulators, stabilizers, preservatives, or fragrances.

The term "sun protection factor (SPF)" as used herein indicates how well the skin is protected by a sunscreen composition mainly from UV-B radiation. In particular, the factor indicates how much longer the protected skin may be exposed to the sun without getting a sunburn in comparison to untreated skin. For example, if a sunscreen composition with an SPF of 15 is evenly applied to the skin of a person usually getting a sunburn after 10 minutes in the sun, the sunscreen allows the skilled person to stay in the sun 15 times longer. In other words, SPF 15 means that 1/15 of the burning UV radiation will reach the skin, assuming sunscreen is applied evenly at a thick dosage of 2 milligrams per square centimeter (mg/cm$^2$).

The term "UV-filter" as used herein refers to organic or inorganic compounds, which can absorb or reflect UV radiation caused by sunlight. UV-filter can be classified based on their UV protection curve as UV-A, UV-B or broadband filters. In the context of the present application, broadband filters may be listed as UV-A filters, as they also provide UV-A protection. In other words, preferred UV-A filters also include broadband filters.

Furthermore, UV filters can be classified according to their solubility behavior, e.g., water soluble UV filters, oil soluble UV filters, or particulate UV-filters.

Water soluble UV filters have a solubility in water of at least 2 % by weight, preferably at least 3 % by weight, more preferably at least 5 % by weight.

Particulate UV filters can be further divided into organic particulate UV filters and inorganic particulate UV filters. While organic particulate UV filters are based on organic compounds, inorganic particulate UV filters are based on inorganic compounds such as titanium dioxide. In the sunscreen composition, particulate UV filters will be present in particulate form, as their solubility is less than 0.01 % by weight, preferably less than 0.05 % by weight in the sunscreen composition, i.e. in the water and the cosmetic oils contained therein. Preferably, the particulate UV filters have a particle size $D_N50$ determined by light scattering of less than 2000 nm, preferably less than 1000 nm, wherein $D_N50$ refers to the particle size value, where half of the population lies below this value, and half of the population lies above this value, i.e. the median value of the particle size volume distribution.

Hexyl 2-[4-(diethylamino)-2-hydroxybenzoyl]benzoate (INCI diethylamino hydroxybenzoyl hexyl benzoate) is an oil soluble UV-A filter, which has an absorption maximum at 354 nm. Diethylamino hydroxybenzoyl hexyl benzoate has an excellent photo stability for long-lasting protection of the skin and provides an efficient shielding against UV-A I and UV-A II radiation. It is sold under the trade name Uvinul® A Plus by BASF.

Ethylhexyl-2-cyano-3,3-diphenyl-acrylate (INCI octocrylene) is an oil soluble organic UV-B filter with an absorption maximum at 302 nm. It provides a broad UV-B absorbance for balanced UV protection and is known to be an efficient stabilizer for photo-unstable UV filters. Octocrylene is sold by BASF under the tradename Uvinul® N 539 T.

2-Hydroxy-4-methoxybenzophenone (INCI oxybenzone) absorbs UV-A light as well as UV-B light and is therefore a so-called broadband UV filter. Oxybenzone can be applied in sunscreen compositions or daily care cosmetics in an amount of from 1% to 6%. Further, it is sold under the trade names Eusolex 4360, Escalol 567 and Neo-Helipan BB.

2-Ethylhexyl-(2E)-3-(4-methoxyphenyl)acrylate (INCI ethylhexyl methoxy cinnamate) is an odorless and colorless UV-B filter with an absorption maximum at 310 nm. It is a good solvent for other crystalline UV filters. Ethylhexyl methoxy cinnamate is sold under the tradename Uvinul® MC 80 by BASF.

The term "paraben" refers to a class of preservatives used in cosmetic and pharmaceutical compositions. They are commonly used due to their bactericidal and fungicidal properties. The chemical structure of parabens is based on parahydroxybenzoates or esters of parahydroxybenzoic acid, e.g. methylparaben, ethylparaben, propylparaben, butylparaben or heptylparaben.

The definition of "broadband" protection (also referred to as broad-spectrum or broad protection) is based on the "critical wavelength". For broadband coverage, UV-B and UV-A protection must be provided. According to the US requirements, a critical wavelength of at least 370 nm is required for achieving broad spectrum protection. Furthermore, it is recommended by the European Commission that all sunscreen or cosmetic compositions should have an UVA protection factor, which is at least one third of the labelled sun protection factor (SPF), e.g. if the sunscreen composition has an SPF of 30 the UVA protection factor has to be at least 10.

The term "fabric staining" as used herein refers to the observation that sunscreen compositions, in particular those providing good UV-A protection, leave yellow stains on textiles. This is undesired because the sunscreen composition is often in contact with textiles during or after the application to the human skin, so that stains will be observed on the textiles. Staining of fabrics can be measured by the L*a*b as outlined in detail below.

The term "washability" as used herein describes to which extent stains on textiles caused by sunscreen compositions can be removed from the textile after a certain number of washing cycles. In order to assess the washability, fabric staining is measured before and after one or more washing cycles. A washing cycle is carried out at a temperature of from 30°C to 50 °C with a commercially available liquid washing gel. Typically, the difference regarding fabric staining after 1 to 3 washing cycles is determined.

Preferred embodiments regarding the sunscreen or daily care composition according to the present application as well as the uses of said composition are described hereinafter. It is to be understood that the preferred embodiments of the invention are preferred alone or in combination with each other.

As indicated above, the present invention relates in one embodiment to a sunscreen or daily care composition comprising

(i) hexyl 2-[4-(diethylamino)-2-hydroxybenzoyl]benzoate (INCI diethylamino hydroxybenzoyl hexyl benzoate); and
(ii) at least one water soluble UVA filter;

wherein the composition does not comprise 2-hydroxy-4-methoxybenzophenone (INCI oxybenzone) and ethylhexyl-2-cyano-3,3-diphenyl-acrylate (INCI octocrylene).

In a second embodiment, the present invention relates to a sunscreen or daily care composition comprising

(i) hexyl 2-[4-(diethylamino)-2-hydroxybenzoyl]benzoate (INCI diethylamino hydroxybenzoyl hexyl benzoate); and
(ii) at least one water soluble UVA filter selected from disodium phenyl dibenzimidazole tetrasulfonate in an amount of from 1% to 10% by weight based on the total weight of the composition;

wherein the composition does not comprise 2-hydroxy-4-methoxybenzophenone (INCI oxybenzone), ethylhexyl-2-cyano-3,3-diphenyl-acrylate (INCI octocrylene) and 2-ethylhexyl-(2E)-3-(4-methoxyphenyl)acrylate (INCI ethylhexyl methoxy cinnamate).

In connection with the present invention, in particular in connection with the first and second embodiment as defined above the following preferences regarding the sunscreen composition are relevant.

In one embodiment of the present invention, the sunscreen or daily care composition comprises

(i) hexyl 2-[4-(diethylamino)-2-hydroxybenzoyl]benzoate (INCI diethylamino hydroxybenzoyl hexyl benzoate); and
(ii) at least one water soluble UVA filter;

wherein the composition does not comprise 2-hydroxy-4-methoxybenzophenone (INCI oxybenzone) and ethylhexyl-2-cyano-3,3-diphenyl-acrylate (INCI octocrylene).

In another embodiment of the present invention, the sunscreen or daily care composition as defined above is free of parabens.

In yet another embodiment of the present invention, the sunscreen or daily care composition as defined above does not comprise 2-ethylhexyl-(2E)-3-(4-methoxyphenyl)acrylate (INCI ethylhexyl methoxy cinnamate).

In yet another embodiment of the present invention, the sunscreen or daily care composition as defined above is free of phenoxyethanol.

In yet another embodiment of the present invention, the sunscreen or daily care composition as defined above is free of (RS)-2-ethylhexyl-2-hydroxybenzoate (INCI ethylhexyl salicylate) and 3,3,5-trimethyl-cyclohexyl salicylate (homosalate).

In yet another embodiment of the present invention, the sunscreen or daily care composition as defined above, may optionally be free of ethoxylated emulsifier. In this connection it is to be understood that the term "emulsifiers", which are also known as emulgents, typically refers to compounds, which stabilize an emulsion by increasing its kinetic stability. In principle, they consist of a polar or hydrophilic part and a non-polar or hydrophobic part.

Possible emulsifiers, which may optionally be excluded from the sunscreen or daily care composition are PEG-100 stearate, steareth derived emulsifier, or ceteareth derived emulsifier.

In connection with the above embodiments, it is to be understood that free of means that the composition does not comprise the above-defined compounds or substances. In particular, it is to be understood that the composition does not comprise the above-defined compounds or substances in an amount of more than 0.8% by weight respectively, based on the total weight of the composition. Furthermore, it is to be understood that the composition does not comprise the above-defined compounds or substances in an amount of more than 0.5% by weight respectively.

In a preferred embodiment of the present invention, the sunscreen or daily care composition as defined above does not comprise each of the compounds or substances as defined above at all.

In connection with the above embodiments, it is to be understood that the sunscreen or daily care composition as defined above according to the present invention is free of the substances as defined above or the combinations as defined below.

Thus, in one preferred embodiment of the present invention, the sunscreen or daily care composition as defined above is free of

- parabens, and
- ethylhexyl methoxy cinnamate.

In another preferred embodiment of the present invention, the sunscreen or daily care composition as defined above is free of

- parabens, and
- phenoxyethanol.

In yet another preferred embodiment of the present invention, the sunscreen or daily care composition as defined above is free of

- ethylhexyl methoxy cinnamate, and
- phenoxyethanol.

In yet another preferred embodiment of the present invention, the sunscreen or daily care composition as defined above is free of

- parabens,
- ethylhexyl methoxy cinnamate, and
- phenoxyethanol.

In yet another preferred embodiment of the present invention, the sunscreen or daily care composition as defined above is free of

- parabens,
- ethylhexyl methoxy cinnamate,
- phenoxyethanol, and
- ethylhexyl salicylate and homosalate.

In connection with the above preferred embodiments, it is to be understood that free of means that the composition does not comprise the substances or combinations thereof as defined above. In particular, it is to be understood that the composition does not comprise the above-defined substances or combinations thereof in an overall amount of more than 0.8% by weight, based on the total weight of the composition. Furthermore, it is to be understood that the composition does not comprise the above-defined substances or combinations thereof in an overall amount of more than 0.5% by weight.

In a preferred embodiment of the present invention, the sunscreen or daily care composition does not comprise the above-defined substances or combinations thereof at all.

Furthermore, in connection with the above preferred embodiments, it is to be understood that the sunscreen or daily care composition does not comprise the substances as defined above or the combinations thereof as defined above, in addition to oxybenzone and octocrylene.

In a particularly preferred embodiment of the present invention, the sunscreen or daily care composition as defined above does not comprise ethylhexyl methoxy cinnamate in addition to oxybenzone and octocrylene.

In connection with the present invention, in particular in connection with the first and second embodiment of the present invention, the following preferences regarding the UV filters are relevant in connection with the above listed embodiments of the invention.

In one embodiment of the present invention, the sunscreen or daily care composition comprises at least one water soluble UVA filter. In this context, at least one water soluble UVA filter means from 1 to 3 water soluble UVA filter. Furthermore, it is to be understood that the term water soluble UVA filter means any UV filter, which have a solubility in water of at least 2% by weight and absorb in the UVA range.

In a preferred embodiment of the present invention, the at least one water soluble UVA filter is selected from the group consisting of [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1-bicyclo[2.2.1]heptanyl]methanesulfonic acid (INCI terephthalylidene dicamphor sulfonic acid), disodium phenyl dibenzimidazole tetrasulfonate, and the combination thereof.

In a particularly preferred embodiment of the present invention, the at least one water soluble UVA filter is disodium phenyl dibenzimidazole tetrasulfonate.

In another embodiment of the present invention, the sunscreen or daily care composition comprises at least one further UVA filter selected from the group consisting of triazine derivatives, dibenzoylmethane derivatives, benzotriazole derivatives and combinations thereof. In this context, at least one further UVA filter means from 1 to 3 further UVA filters, preferably 1 or 2 further UVA filters. Furthermore, it is to be understood that the term UVA filter means any UV filter absorbing in the UVA range. This means that also broadband filters are encompassed by the expression UVA filter, since they absorb and/or reflect UVA and UVB radiation.

In a more preferred embodiment, the at least one further UVA filter is selected from the group consisting of 4-(tert.-butyl)-4'-methoxydibenzoylmethane (INCI butyl methoxydibenzoylmethane), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane), 2-(2H-benzotriazol-2-yl)-6-( 2-ethylhexyloxymethyl)-4-methyl-phenol and combinations thereof.

In this context, the at least one further UVA filter refers to UVA filter which are not water soluble UVA filter as defined above.

In another embodiment of the present invention, the sunscreen or daily care composition further comprises at least one UVB filter. In this context, at least one UVB filter means from 1 to 3 UVB filters. Furthermore, it is to be understood that the term UVB filter means any UV filter absorbing in the UVB range. This means that also broadband filters are encompassed by the expression UVB filter, since they absorb and/or reflect UVA and UVB radiation.

In a preferred embodiment, the at least one further UVB filter is selected from the group consisting of 4,4',4"-(1 ,3,5-triazin-2,4,6-triyltriimino)tris-benzoic acid-tris(2-ethylhexyl)ester (INCI ethylhexyl triazone), malonate derivatives such as dimethicone diethyl benzalmalonate, diethylhexyl butamido triazone, and combinations thereof.

Suitable amounts of water soluble UVA filter are in the range of from 0,1% to 10% by weight, preferably in the range of from 0.3% to 5% by weight, even more preferably in the range of from 0.5% to 3% by weight, based on the total weight of the composition.

In a preferred embodiment of the present invention, suitable amounts of water soluble UVA filter are in the range of from 1 % to 10% by weight based on the total weight of the composition.

In a more preferred embodiment of the present invention, suitable amounts of water soluble UVA filter are in the range of from 1% to 8% by weight, preferably in an amount of from 1% to 7% by weight, more preferably in an amount of from 1% to 6% by weight based on the total weight of the composition.

Suitable amounts of a further UVA filter, which is not a water soluble UVA filter, are in the range of from 0.5% to 5% by weight, based on the total weight of the composition.

Suitable amounts of a further UVB filter are in the range of from 0.5% to 5% by weight, based on the total weight of the composition.

In connection with the above embodiments, it is to be understood that the at least one water soluble UVA filter as defined above, is present in the sunscreen or daily care composition in addition to hexyl 2-[4-(diethylamino)-2-hydroxybenzoyl]benzoate (INCI diethylamino hydroxybenzoyl hexyl benzoate). Furthermore, it is to be understood that the sunscreen composition comprises hexyl 2-[4-(diethylamino)-2-hydroxybenzoyl]benzoate (INCI diethylamino hydroxybenzoyl hexyl benzoate) and at least one water soluble UVA filter and may optionally comprise at least one further UVA filter and at least one UVB filter.

In a preferred embodiment of the present invention, the sunscreen or daily care composition comprises hexyl 2-[4-(diethylamino)-2-hydroxybenzoyl]benzoate (INCI diethylamino hydroxybenzoyl hexyl benzoate) in an amount of from 1% to 10% by weight, based on the total weight of the composition.

In a particularly preferred embodiment of the present invention, the sunscreen or daily care composition comprises

(i) hexyl 2-[4-(diethylamino)-2-hydroxybenzoyl]benzoate (INCI diethylamino hydroxybenzoyl hexyl benzoate); and
(ii) at least one water soluble UVA filter, which is [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1  -bicyclo[2.2.1]heptanyl]methanesulfonic acid (INCI terephthalylidene dicamphor sulfonic acid) and/or disodium phenyl dibenzimidazole tetrasulfonate;

wherein the composition does not comprise 2-hydroxy-4-methoxybenzophenone (INCI oxybenzone) and ethylhexyl-2-cyano-3,3-diphenyl-acrylate (INCI octocrylene); and wherein the composition is free of parabens.

[(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1 - bicyclo[2.2.1]heptanyl]methanesulfonic acid (INCI terephthalylidene dicamphor sulfonic acid) is a water soluble organic UVA filter, which absorbs UVA radiation in the range of from 320 to 400 nm.

Disodium phenyl dibenzimidazole tetrasulfonate is a water soluble organic UVA filter, which absorbs UVA radiation in the range of from 320 to 400 nm.

In connection with the above preferred and particularly preferred embodiments, it has surprisingly been found that the water soluble UVA filter terephthalylidene dicamphor sulfonic acid is instable upon irradiation with UV light. Furthermore, it has surprisingly been found that in the sunscreen or daily care composition according to the present invention the water soluble UV filter terephthalylidene dicamphor sulfonic acid can be stabilized in the presence of the UV filter diethylamino hydroxybenzoyl hexyl benzoate.

In connection with the above embodiments, it is to be understood that the total weight of the sunscreen or daily care composition refers to the composition as defined above, wherein the sunscreen composition may comprise at least one additive.

In one embodiment, the at least one additive is selected from the group consisting of emulsifier, emollients, viscosity regulators (thickeners), sensory enhancers, adjuvants, preservatives, and combinations thereof.

Preferred emulsifiers include

- glucose derivatives such as cetearyl glucoside, arachidyl glucoside, lauryl glucoside, polyglyceryl-3 methylglucose distearate, methyl glucose sesquistearate;
- sucrose derivative such as sucrose polystearate, sucrose palmitate;
- sorbitol derivatives;
- glycerides of fatty acids such as glyceryl stearate, glyceryl oleate;
- glumatic acid derivatives such as sodium stearoyl glutamate;
- sulfosuccinic acid derivatives such as disodium cetearyl sulfosuccinate;
- phosphoric acid derivatives such as potassium cetyl phosphate;
- fatty acid esters of polyglyceryl such as polyglyceryl-3-diisostearate, polyglyceryl-2-dipolyhydroxystearate;
- oxyalkenylated organomodified silicone / polysiloxane / polyalkyl / polyether copolymers and derivatives.

Preferred emollients include

- esters of linear or branched fatty acids with linear or branched fatty alcohols such as propylheptyl caprylate, coco caprylate, isopropyl myristate, ethylhexyl palmitate;
- esters of aromatic carboxylic acids with linear or branched fatty alcohols such as $C_{12}$-$C_{15}$-alkyl benzoate, ethylhexyl benzoate, phenethyl benzoate;
- di- and tricarboxylic acid esters with linear or branched alcohols such as dibutyl adipate, dicaprylyl carbonate, diisopropyl sebacate, triethyl citrate, tributyl citrate;
- esters of hydroxycarboxylic acids with linear or branched fatty alcohols;
- esters of linear or branched fatty acids with polyhydric alcohol such as butylene glycol dicaprylate/dicaprate;
- mono-, di-, tri-glycerides based on $C_6$-$C_{18}$ fatty acids such as caprylic / capric triglycerides, coco glycerides;
- guerbet alcohols such as octyldodecynol;
- hydrocarbons such as hydrogenated polyisobutene, mineral oil, squalene, isohexadecane;
- ethers such as dicaprylyl ether;
- silicone derivatives (organomodified polysiloxanes) such as dimethylpolysiloxane, cyclic silicones.

Preferred thickeners include

- fatty alcohols such as cetyl alcohol, cetearyl alcohol, stearyl alcohol;
- fatty acids such as stearic acid;
- fatty acid esters such as myristyl stearate;
- waxes such as beeswax, carnauba wax, microcrystalline wax, ceresin, ozocerite;
- polysaccharides or derivatives such as xanthan gum, guar gum, agar gum, alginates, gellan gum, carraghenan;
- polyacrylates or homopolymers of reticulated acrylic acids or polyacrylamides such as carbomers, acrylate copolymers, acrylate / $C_{10}$-$C_{30}$-alkyl acrylate crosspolymer, acrylate / beheneth-25 methacrylate copolymer;
- silicate derivatives such as magnesium silicates;
- cellulose derivatives such as hydroxypropyl cellulose.

Preferred sensory enhancers include

- polyamide derivatives such as nylon-12;
- polymethyl methacrylates;
- silica;
- mica;
- polymethylsilsesquioxane;

- polyethylene;
- starch derivatives such as aluminum starch octenylsuccinate;
- dimethicone derivatives;
- boron nitride;
- HDI / trimethylol hexyllactone crosspolymer.

Preferred adjuvants include

- tocopherol derivatives;
- retinol derivatives;
- ascorbic acid derivatives;
- bisabolol;
- allantoin;
- panthenol;
- chelating agents (EDTA, EDDS, EGTA, phytic acid, piroctone olamine);
- ethylhexyl glycerin;
- caprylyl glycol;
- hydroxyacetophenone;
- caprylhydroxymic acid;
- propellants such as propane, butane, isobutene, dimethyl ether;
- styrene / PVP or styrene acrylamide copolymers;
- insect repellants such as butylacetylaminopropionate.

Preferred preservatives include

- benzyl alcohol;
- zingerone.

In a preferred embodiment of the present invention, the sunscreen or daily care composition comprises at least one perfume.

Preferred perfumes are selected from the group consisting of limonene, citral, linalool, alpha-isomethylionon, geraniol, citronellol, 2-isobutyl-4-hydroxy-4-methyltetrahydropyrane, 2-tert.-pentylcyclohexylacetate, 3-methyl-5-phenyl-1-pentanol, 7-acetyl-1,1,3,4,4,6-hexamethyltetraline, adipine acid diester, alpha-amylcinnamaldehyde, alpha-methylionon, amyl C butylphenylmethylpropionalcinnamal, amylsalicylate, amylcinnamylalcohol, anisalcohol, benzoin, benzylalcohol, benzylbenzoate, benzylcinnamate, benzylsalicylate, bergamot oil, bitter orange oil, butylphenylmethylpropioal, cardamom oil, cedrol, cinnamal, cinnamylalcohol, citronnellylmethylcrotonate, lemon oil, coumarin, diethylsuccinate, ethyllinalool, eugenol, evernia furfuracea extracte, evernia prunastri extracte, farensol, guajak wood oil, hexylcinnamal, hexylsalicylate, hydroxycitronellal, lavender oil, lemon oil, linaylacetate, mandarine oil, menthyl PCA, methylheptenone, nutmeg oil, rosemary oil, sweet orange oil, terpineol, tonka bean oil, triethylcitrate, vanillin and combinations thereof.

In connection with the at least one perfume present in the sunscreen or daily care composition, it has surprisingly been found that in case the composition comprises a UV filter in combination with a perfume, oxidative degeneration is reduced. In particular, it has surprisingly been found that the UV filter DHHB in the presence of at least one perfume reduces the oxidative degeneration.

In connection with the above preferred embodiments, it is to be understood that if the sunscreen or daily care composition comprises two or more additives, combinations of the additives as defined above are also part of the invention.

In connection with the above preferred and particularly preferred embodiments, it is to be understood that the sunscreen or daily care composition, in its final formulation, may exist in a wide variety of presentation forms, which include

- liquid preparations as a W/O, O/W, O/W/O, W/O/W or PIT emulsion and all kinds of micro emulsions;
- gels;
- oil, cream, milk or lotion;
- powder, a lacquer, a tablet or make-up;
- sticks;
- sprays (spray with propellant gas or pump-action spray) or an aerosol;
- foams;
- pastes.

As indicated above, the present invention relates in another aspect to the use of a sunscreen or daily care composition as defined above, in particular as defined in the first and second embodiment, to reduce fabric staining of a sunscreen or daily care composition.

Fabric staining caused by sunscreen compositions is preferably measured by the so-called L*a*b method. With this method, the color difference between two samples can be identified based on numerical values. The advantage of this method is that it is not dependent on the device used for measuring the colors. Instead, the color difference can be determined based on numerical values for different colors. As used herein, the color difference between a sunscreen composition stain on a textile and the textile alone is determined in order to determine fabric staining of a sunscreen composition. Different sunscreen compositions and their fabric staining properties can thus be measured by comparing the color of their stains on a textile with the color of the textile alone. The lower the color difference is, the less fabric staining is observed. Similarly, the color difference between a sunscreen composition stain on a textile after one or more washing cycles and the textile alone can be determined in order to determine a potential reduction of fabric staining after washing, i.e. the washability. The color difference is indicated in absolute color coordinates and can be referred to as Delta $\Delta$. First, the colors of the two samples are measured. Then, the difference can be calculated using the resulting colorimetric values, the CIE L*a*b coordinates. The L*a*b color space, defined by the Commission Internationale de l'Eclairage (CIE), was modeled after a color-opponent theory. This theory states that two colors cannot be red and green at the same time or yellow and blue at the same time. The following formula is used to determine the total color difference between two samples:

$$\Delta E^* = [\Delta L^{*2} + \Delta a^{*2} + \Delta b^{*2}]1/2$$

In this regard, L* indicates lightness, a* is the red/green coordinate, and b* is the yellow/blue coordinate. Delta values for L* ($\Delta L^*$), a* ($\Delta a^*$), and b* ($\Delta b^*$) can be positive or negative. The total difference, given as $\Delta E^*$ is always positive.

$$\Delta L^* \ (L^* \text{ sample1} - L^* \text{ sample 2}) = \text{difference in lightness and darkness (+ is lighter, - is darker).}$$

$$\Delta a^* \ (a^* \text{ sample 1} - a^* \text{ sample 2}) = \text{difference in red and green (+ is redder, - is greener)}$$

$$\Delta b^* \ (b^* \text{ sample 1} - b^* \text{ sample 2}) = \text{difference in yellow and green (+ is yellower, - is greener)}$$

$$\Delta E^* = \text{total color difference.}$$

In order to determine fabric staining according to the present invention, the color of the stain on a textile is compared to the color of the textile alone. Preferably, a white textile is used. In case the stain is only yellow, $\Delta E^* \cong \Delta b^*$, wherein the visibility of the sunscreen composition stain is expressed by $\Delta b^*$. As the color of a sunscreen composition stain is typically yellow, $\Delta E^*$ is considered to be identical to $\Delta b^*$ for the purpose of the present invention.

In another aspect, the present invention relates to the use of a sunscreen or daily care composition as defined above, in particular as defined in the first and second embodiment, to facilitate the washability of a sunscreen or daily care composition from textiles.

As used herein, the washability refers to the extent to which sunscreen composition stains on textiles can be removed by washing. The washability, i.e. the removability, of a sunscreen composition stain can be determined by determining the color difference of the stain according to the L*a*b* method described above before and after one or more washing cycles.

The present invention is further illustrated by the following examples.

Examples

Process of manufacture of sunscreen compositions

The ingredients of part A, as well as the ingredients of part B as provided below in Table 1, for each tested sunscreen composition were combined and heated to 80 °C respectively, wherein part A was added to part B under stirring and was further homogenized. If present, phase C was premixed in an ultra turrax device and added to phase A and B. Subsequently, the sunscreen composition was cooled down to room temperature under stirring and the pH of each composition was adjusted to > 7.00.

All amounts referred to in the following tables refer to the respective amounts in % by weight, based on the total weight of the composition.

Sunscreen compositions

Table 1

| Ingredient (Trade Name) | Comparative composition 1 | Composition 1 |
|---|---|---|
| Part A | | |
| Dibutyl adipate (Cetiol B) | 10.00 | 10.00 |
| $C_{12}$-$C_{15}$ alkyl benzoate (Cetiol AB) | 10.00 | 10.00 |
| Stearyl alcohol (Lanette 18) | 2.50 | 2.50 |
| DHHB (Uvinul A Plus) | 5.00 | 2.50 |
| EHT (Uvinul T 150) | 3.00 | 3.00 |
| Part B | | |
| Water | qsp 100% | qsp 100% |
| Glycerin | 2.00 | 2.00 |
| Disodium EDTA (EDTA BD) | 0.20 | 0.20 |
| Acrylates/Beheneth-25 methacrylate copolymer (Tinovis GTC UP) | 2.00 | 2.00 |
| Xanthan Gum (Rheocare XGN) | 0.50 | 0.50 |
| Part C | | |
| Water | - | 5.00 |
| Tromethamine (Tris Amino) | - | qs |
| DPDT (Neoheliopan AP) | - | 2.5 |

The calculated SPF for the comparative composition is 12.
The calculated SPF for composition 1 is 17.

Sunscreen compositions

The ingredients of part A, as well as the ingredients of part B as provided below in Table 2, for each tested sunscreen composition were combined and heated to 80 °C respectively, wherein part A was added to part B under stirring and was further homogenized. If present, phase C was premixed in an ultra turrax device and added to phase A and B. Subsequently, the sunscreen composition was cooled down to room temperature, then add part D under stirring, finally add part E when present under stirring and adjust the pH of each composition to > 7.00.

Table 2

| INCI | Comparativ e Composition 2 | Composition 2 | Comparativ e Composition 3 | Composition 3 |
|---|---|---|---|---|
| Part A | | | | |
| Glyceryl stearate | 1.00 | 1.00 | 1.00 | 1.00 |
| Sodium Stearyl Glutamate | 0.30 | 0.30 | 0.30 | 0.30 |
| Stearyl Alcohol | 1.00 | 1.00 | 1.00 | 1.00 |
| Butylene Glycol Dicaprylate/Dicaprate | 4.00 | 4.00 | 4.00 | 4.00 |
| Dibutyl adipate | 3.00 | 3.00 | 3.00 | 3.00 |

(continued)

| INCI | Comparativ e Composition 2 | Composition 2 | Comparativ e Composition 3 | Composition 3 |
|---|---|---|---|---|
| Part A | | | | |
| C12-15 Alkyl Benzoate | 5.00 | 5.00 | 5.00 | 5.00 |
| Phenoxyethanol | 1.00 | 1.00 | 1.00 | 1.00 |
| VP/Hexadecene Copolymer | 0.50 | 0.50 | 0.50 | 0.50 |
| Silica Dimethyl Silylate | 1.00 | 1.00 | 1.00 | 1.00 |
| Butyl Methoxy Diben-zoylmethane | 4.75 | | 4.75 | 4.75 |
| Ethylhexyl Salicylate | 4.75 | 4.75 | 4.75 | 4.75 |
| Ethylhexyl Triazone | 3.00 | 1.50 | 1.50 | 1.00 |
| Bis-ethylhexyloxyphe-nol Methoxyphenyl Triazine | 4.00 | 4.00 | | |
| Diethylamino Hydroxybenzoyl Hexyl Ben-zoate | 1.00 | 1.00 | 1.00 | 1.00 |
| Part B | | | | |
| Aqua | qsp 100 | qsp 100 | qsp 100 | qsp 100 |
| Glycerin | 7.50 | 7.50 | 7.50 | 7.50 |
| Acrylates/C10-30 Alkyl Acrylate Crosspolymer | 0.10 | 0.10 | 0.10 | 0.10 |
| Xanthan gum | 0.40 | 0.40 | 0.40 | 0.40 |
| Dissodium EDTA | 0.20 | 0.20 | 0.20 | 0.20 |
| Part C | | | | |
| Phenylbenzimidazol e Sulfonic Acid | 1.00 | | 1.00 | 1.00 |
| Disodium Phenyl Dibenzimidazole Tetrasulfonate | | 4.75 | | 7.00 |
| Water | 5.00 | 5.00 | 5.00 | 5.00 |
| Sodium Hydroxide | qsp | qsp | qsp | qsp |
| Part D | | | | |
| Ethanol | 3.00 | 3.00 | 3.00 | 3.00 |
| Part E | | | | |
| Bis-ethylhexyloxy-phenol Methoxyphenyl Triazine (Tinosorb S Lite Aqua)* | | | 20.00 | |
| * Tinosorb S Lite Aqua is a 20% aqueous dispersion of Bis-ethylhexylphenol Methoxyphenyl Triazine; 20% Tinosorb S Lite Aqua corresponds to 4% Bis-ethylhexyloxyphenol Methoxyphenyl Triazine | | | | |

Measurement of fabric staining

Fabric staining of UV filter compositions was measured by the L*a*b as described above. The measurement was performed with a spectrophotometer using the following instrument: Remission Spectrometer Datacolor SF 400 (light D65, observer 10°).

The difference between the color of a UV filter composition stain on a white textile and the color of the white textile alone was determined following the assumption that $\Delta E^* = \Delta b^*$ in order to determine fabric staining. The lower the color difference, the lower is the fabric staining.

Further, the difference between the color of a UV filter composition stain on a white textile after 1 or 3 washing cycles and the color of the white textile alone was determined following the assumption that ΔE* = Δb* in order to determine the washability. The lower the color difference in comparison to the value obtained before washing, the more has fabric staining been reduced, i.e. the better the washability of the sunscreen composition from textiles.

For applying the UV filter compositions in order to make a stain on a white textile, the UV filter composition was diluted in water or ethanol at a concentration of 10 % by weight based on the total weight of the diluted mixture. 300 mg (15 mg/cm$^2$) of the diluted mixture is applied on a white textile (whiteness adjusted to WG 170 ± 10, after washing WG 190 ± 10) in order to obtain a stain.

For determining the washability, 1 or 3 washing cycles were performed in a washing machine using a temperature of 40 °C for 20 minutes and Persil Universal Gel obtained from Henkel (3 g on stain). Each textile was washed separately.

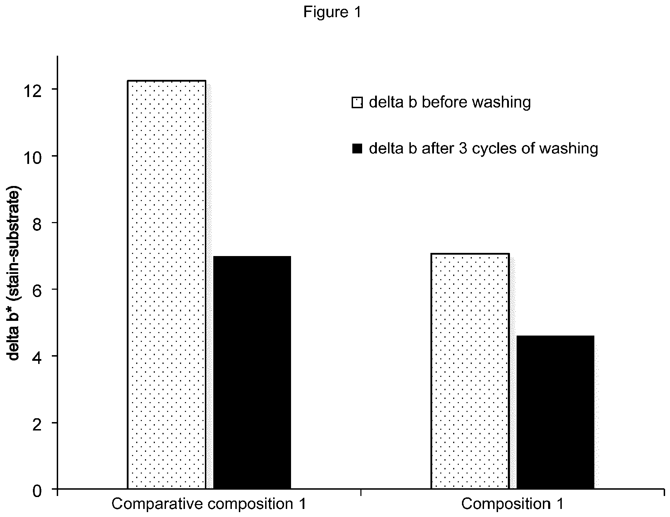

The results for the comparative composition 1 and composition 1 are shown in Table 3 and Figure 1.

The results of comparative compositions 2, 3 and compositions 2, 3 are shown in Table 4.

Table 3

|  | Comparative composition 1 | Composition 1 |
|---|---|---|
| Δb* (before washing) | 12.25 | 7.06 |
| SD (before washing) | 0.22 | 0.17 |
| Δb* (after 3 cycles) | 6.99 | 4.61 |
| SD (after 3 cycles) | 0.01 | 0.09 |

Table 4

|  | Comparative Composition 2 | Composition 2 | Comparative Composition 3 | Composition 3 |
|---|---|---|---|---|
| Δb* (before washing) | 10.91 | 4.94 | 11.29 | 6.15 |
| SD (before washing) | 0.04 | 0.28 | 0.05 | 0.22 |
| Δb* (after 3 cycles) | 3.90 | 2.93 | 4.06 | 2.23 |
| SD (after 3 cycles) | 0.09 | 0.26 | 0.08 | 0.34 |

As can be seen from the results above, comparative Composition 2 containing only oil soluble UVA filters exhibts a strong fabric staining. Composition 2 containing DPDT instead of BMBDM shows a significant fabric staining reduction. Comparative Composition 3 containing Tinosorb S Lite Aqua as a water dispersed UV filter instead of DPDT shows that this UVA filter present in the water phase is not appropriate for fabric staining reduction. Composition 3 containing both DHHB and BMBDM and DPDT shows fabric staining reduction.

In a further step, the following filter combinations have been tested to show that not every UV filter present in the water phase is suitable to contribute to reduction of fabric staining. Table 5 shows a test series with various UV filters present in the water and oil phase incorporated in emulsions and their performance in reducing fabric staining.

Table 5

| Test Emulsion | Test emulsion 1 | Test emulsion 2 | Test emulsion 3 | Test emulsion 4 |
|---|---|---|---|---|
| Part A |  |  |  |  |
| Dibutyl Adipate | 10.00 | 10.00 | 10.00 | 10.00 |
| C12-15 Alkyl Benzoate | 10.00 | 10.00 | 10.00 | 10.00 |
| Stearyl Alcohol | 2.50 | 2.50 | 2.50 | 2.50 |
| Diethylamino Hydroxybenzoyl Hexyl Benzoate | 3.00 |  | 1.50 | 1.50 |
| Ethylhexyl Triazone | 3.50 | 1.20 | 2.30 | 2.30 |
| Part B |  |  |  |  |

(continued)

| Test Emulsion | Test emulsion 1 | Test emulsion 2 | Test emulsion 3 | Test emulsion 4 |
|---|---|---|---|---|
| Aqua | qsp 100 | qsp 100 | qsp 100 | qsp 100 |
| Glycerin | 2.00 | 2.00 | 2.00 | 2.00 |
| Disodium EDTA | 0.20 | 0.20 | 0.20 | 0.20 |
| Acrylates/Beheneth-25 Methacrylate Copolymer (Tinovis GTC)* | 2.00 | 2.00 | 2.00 | 2.00 |
| Xanthan Gum | 0.50 | 0.50 | 0.50 | 0.50 |
| Part C | | | | |
| Aqua | | 5.00 | 5.00 | |
| Neutralizing agent | | qs | qs | |
| Disodium Phenyl Dibenzimidazole Tetrasulfate | | 5.00 | 1.80 | |
| Part D | | | | |
| Bis-ethylhexyloxyphenol Methoxyphenyl Triazine (Tinosorb S Lite Aqua) ** | | | | 7.50 |
| | | | | |
| SPF calculated | 15.5 | 15.3 | 14.9 | 15.0 |
| UVA-PF calculated (SPF15) | 5.7 | 5.7 | 5.8 | 6.2 |
| UVA-PF measured (SPF 15) | 5.0 | 3.7 | 4.7 | 5 |

*Tinovis GTC is a 30% polymer dispersion in water.
**Tinosorb S Lite Aqua is a 20% aqueous dispersion of Bis-ethylhexyloxyphenol Methoxyphenyl Triazine; 7.5% Tinosorb S Lite Aqua corresponds to 1,5% Bis-ethylhexyloxyphenol Methoxyphenyl Triazine.

The test emulsions are prepared as follows: Part A and B are heated to 80°C under stirring. Part A is added into B under stirring and homogenized. When present phase C premixed under ultra turrax device is added. It is cooled down to room temperature. When present phase D is added. The pH is checked to be > 7.

The results obtained from fabric staining tests are shown in Table 6.

Table 6. Fabric staining results

| | Test emulsion 1 | Test emulsion 2 | Test emulsion 3 | Test emulsion 4 |
|---|---|---|---|---|
| $\Delta b^*$ (before washing) | 9.5 | -0.85 | 5.29 | 8.60 |
| SD (before washing) | 0.05 | 0.06 | 0.23 | 0.13 |
| $\Delta b^*$ (after 3 cycles) | 3.31 | 0.38 | 2.06 | 2.68 |
| SD (after 3 cycles) | 0.06 | 0.02 | 0.07 | 0.01 |

Test emulsion 1 contains an oil soluble UVA filter resulting in fabric staining, which is not appreciated by the consumers. Conversely, Test emulsion 2 contains only UVA water soluble filter showing no fabric staining but lower UVA protection than required (UVA-PF measured was equal to 3.7 instead of expected 5.7 (Table 5). The inventive UV filter combination DHHB and DPDT (oil soluble and water soluble) brings about a reduction in fabric staining in test emulsion 3 by simultaneously keeping the UVA performance. In test emulsion 4 DPDT is replaced by Tinosorb S Lite Aqua which is a water dispersed UVA filter. There is no positive effect on fabric staining and washability.

Recovery measurements on photo stability of TDSA

Process of manufacture of sunscreen compositions

The ingredients of part A, as well as the ingredients of part B as provided below in Table 7, for each tested sunscreen composition were combined and heated to 80 °C respectively, wherein part A was added to part B under stirring and was further homogenized. If present, phase C was premixed in an ultra turrax device and added to phase A and B. Subsequently, the sunscreen composition was cooled down to room temperature under stirring and the pH of each composition was adjusted to > 7.00.

Sunscreen compositions

Table 7

| Ingredient (Trade Name) | Comparative composition 4 | Composition 4 |
|---|---|---|
| Part A | | |
| Sucrose polystearate (and) hydrogenated polyisobutene (Emulgade Sucro) | 3 | 3 |
| Disodium cetearyl sulfosuccinate (Emulgin Prisma) | 1 | 1 |
| Dibutyl adipate (Cetiol B) | 10 | 10 |
| Diisopropyl sebacate | 4 | 4 |
| Cetearyl alcohol (Lanette O) | 1 | 1 |
| DHHB (Uvinul A Plus) | - | 6.00 |
| Part B | | |
| Water | qsp 100% | qsp 100% |
| Glycerin | 3.00 | 3.00 |
| Disodium EDTA (EDTA BD) | 0.20 | 0.20 |
| Xanthan Gum (Rheocare XGN) | 0.50 | 0.50 |
| Part C | | |
| Tromethamine (Tris Amino) | qs | qs |
| TDSA* | 2.00 | 2.00 |
| *Pre-dispersion in water, 2% corresponds to the active amount. | | |

The sunscreen compositions as defined above are applied on roughened quartz plates (2 $\mu$l/cm$^2$). Plates are irradiated using Atlas CPS device at different duration times (MED = Minimal Erythema Dose):

- 0h (0 MED, no irradiation)
- 2h (10 MED),
- 10h (50 MED).

In total, four plates are prepared for each irradiation condition. After irradiation, each plate is rinsed off with tetrahydrofuran. The rinsing solution is further analyzed via HPLC to determine the recovery of TDSA.

Table 8

| | Comparative composition 4 | Composition 4 |
|---|---|---|
| 0 MED | 100% | 100% |
| 10 MED | 88% | 93% |

17

(continued)

|  | Comparative composition 4 | Composition 4 |
| --- | --- | --- |
| 50 MED | 60% | 77% |

In a particularly preferred embodiment, the present invention relates to the following further items.

1. A sunscreen or daily care composition comprising

(i) hexyl 2-[4-(diethylamino)-2-hydroxybenzoyl]benzoate (INCI diethylamino hydroxybenzoyl hexyl benzoate); and
(ii) at least one water soluble UVA filter;

wherein the composition does not comprise 2-hydroxy-4-methoxybenzophenone (INCI oxybenzone) and ethylhexyl-2-cyano-3,3-diphenyl-acrylate (INCI octocrylene).

2. The sunscreen or daily care composition according to item 1, wherein the composition is free of parabens.

3. The sunscreen or daily care composition according to item 1 or 2, wherein the at least one water soluble UVA filter is selected from the group consisting of [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1-bicyclo[2.2.1]heptanyl]methanesulfonic acid (INCI terephthalylidene dicamphor sulfonic acid), disodium phenyl dibenzimidazole tetrasulfonate, and the combination thereof.

4. The sunscreen or daily care composition according to any one of items 1 to 3, wherein the composition comprises at least one further UVA filter selected from the group consisting of triazine derivatives, dibenzoylmethane derivatives , benzotriazole derivatives and combinations thereof.

5. The sunscreen or daily care composition according to item 4, wherein the at least one further UVA filter is selected from the group consisting of 4-(tert.-butyl)-4'-methoxydibenzoylmethane (INCI butyl methoxydibenzoylmethane), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane), 2-(2H-benzotriazol-2-yl)-6-(2-ethylhexyloxymethyl)-4-methyl-phenol and combinations thereof.

6. The sunscreen or daily care composition according to any one of items 1 to 5, wherein the composition further comprises at least one UVB filter selected from the group consisting of 4,4',4"-(1,3,5-triazin-2,4,6-triyltriimino)tris-benzoic acid-tris(2-ethylhexyl)ester (INCI ethylhexyl triazone), malonate derivatives such as dimethicone diethyl benzalmalonate, diethylhexyl butamido triazone, and combinations thereof.

7. The sunscreen or daily care composition according to any one of items 1 to 6, wherein the composition does not comprise 2-ethylhexyl-(2E)-3-(4-methoxyphenyl)acrylate (INCI ethylhexyl methoxy cinnamate).

8. The sunscreen or daily care composition according to any one of items 1 to 7, wherein the composition is free of phenoxyethanol.

9. The sunscreen or daily care composition according to any one of items 1 to 8, wherein the composition comprises hexyl 2-[4-(diethylamino)-2-hydroxybenzoyl]benzoate (INCI diethylamino hydroxybenzoyl hexyl benzoate) in an amount of from 1 % to 10 % by weight, based on the total weight of the composition.

10. The sunscreen or daily care composition according to any one of items 1 to 9, wherein the composition comprises the at least one water soluble UVA filter in an amount of from 0,1% to 10% by weight, preferably in an amount of from 0.3% to 5% by weight, more preferably in an amount of from 0.5% to 3% by weight, based on the total weight of the composition.

11. The sunscreen or daily care composition according to any one of items 1 to 10 comprising

(i) hexyl 2-[4-(diethylamino)-2-hydroxybenzoyl]benzoate (INCI diethylamino hydroxybenzoyl hexyl benzoate); and

(ii) at least one water soluble UVA filter, which is [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1   -bicyclo[2.2.1]heptanyl]methanesulfonic acid (INCI terephthalylidene dicamphor sulfonic acid) and/or disodium phenyl dibenzimidazole tetrasulfonate;

wherein the composition does not comprise 2-hydroxy-4-methoxybenzophenone (INCI oxybenzone) and ethylhexyl-2-cyano-3,3-diphenyl-acrylate (INCI octocrylene); and
wherein the composition is free of parabens.

12. The sunscreen or daily care composition according to any one of items 1 to 11, wherein the composition is free of (RS)-2-ethylhexyl-2-hydroxybenzoate (INCI ethylhexyl salicylate) and 3,3,5-trimethyl-cyclohexyl salicylate (homosalate).

13. Use of a sunscreen or daily care composition according to any one of items 1 to 12 to reduce fabric staining.

14. Use of a sunscreen or daily care composition according to any one of items 1 to 12 to facilitate the washability from textiles.

In another particularly preferred embodiment, the present invention relates to the following items.

1. A sunscreen or daily care composition comprising

(i) hexyl 2-[4-(diethylamino)-2-hydroxybenzoyl]benzoate (INCI diethylamino hydroxybenzoyl hexyl benzoate); and
(ii) at least one water soluble UVA filter selected from disodium phenyl dibenzimidazole tetrasulfonate in an amount of from 1% to 10% by weight based on the total weight of the composition;

wherein the composition does not comprise 2-hydroxy-4-methoxybenzophenone (INCI oxybenzone), ethylhexyl-2-cyano-3,3-diphenyl-acrylate (INCI octocrylene) and 2-ethylhexyl-(2E)-3-(4-methoxyphenyl)acrylate (INCI ethylhexyl methoxy cinnamate).

2. The sunscreen or daily care composition according to item 1, wherein the composition is free of parabens.

3. The sunscreen or daily care composition according to item 1 or 2, wherein the composition comprises at least one further UVA filter selected from the group consisting of triazine derivatives, dibenzoylmethane derivatives, benzotriazole derivatives and combinations thereof.

4. The sunscreen or daily care composition according to item 3, wherein the at least one further UVA filter is selected from the group consisting of 4-(tert.-butyl)-4'-methoxydibenzoylmethane (INCI butyl methoxydibenzoylmethane), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl    triazine),    2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane), 2-(2H-benzotriazol-2-yl)-6-(2-ethylhexyloxymethyl)-4-methyl-phenol and combinations thereof.

5. The sunscreen or daily care composition according to any one of items 1 to 4, wherein the composition further comprises at least one UVB filter selected from the group consisting of 4,4',4"-(1,3,5-triazin-2,4,6-triyltriimino)tris-benzoic acid-tris(2-ethylhexyl)ester (INCI ethylhexyl triazone), malonate derivatives such as dimethicone diethyl benzalmalonate, diethylhexyl butamido triazone, and combinations thereof.

6. The sunscreen or daily care composition according to any one of items 1 to 5, wherein the composition is free of phenoxyethanol.

7. The sunscreen or daily care composition according to any one of items 1 to 6, wherein the composition comprises hexyl 2-[4-(diethylamino)-2-hydroxybenzoyl]benzoate (INCI diethylamino hydroxybenzoyl hexyl benzoate) in an amount of from 1 % to 10 % by weight, based on the total weight of the composition.

8. The sunscreen or daily care composition according to any one of items 1 to 7, wherein the composition comprises the at least one water soluble UVA filter in an amount of from 1 % to 8% by weight, preferably in an amount of from 1% to 7% by weight, more preferably in an amount of from 1% to 6% by weight, based on the total weight of the composition.

9. The sunscreen or daily care composition according to any one of items 1 to 8, wherein the composition is free of (RS)-2-ethylhexyl-2-hydroxybenzoate (INCI ethylhexyl salicylate) and 3,3,5-trimethyl-cyclohexyl salicylate (homosalate).

10. The sunscreen or daily care composition according to any one of items 1 to 9, wherein the composition comprises at least one perfume.

11. Use of a sunscreen or daily care composition comprising

(i) hexyl 2-[4-(diethylamino)-2-hydroxybenzoyl]benzoate (INCI diethylamino hydroxybenzoyl hexyl benzoate); and
(ii) at least one water soluble UVA filter;

wherein the composition does not comprise 2-hydroxy-4-methoxybenzophenone (INCI oxybenzone) and ethylhexyl-2-cyano-3,3-diphenyl-acrylate (INCI octocrylene) to reduce fabric staining.

12. Use of a sunscreen or daily care composition comprising

(i) hexyl 2-[4-(diethylamino)-2-hydroxybenzoyl]benzoate (INCI diethylamino hydroxybenzoyl hexyl benzoate); and
(ii) at least one water soluble UVA filter;

wherein the composition does not comprise 2-hydroxy-4-methoxybenzophenone (INCI oxybenzone) and ethylhexyl-2-cyano-3,3-diphenyl-acrylate (INCI octocrylene) to facilitate the washability from textiles.

**Claims**

1.  A sunscreen or daily care composition comprising

(i) hexyl 2-[4-(diethylamino)-2-hydroxybenzoyl]benzoate (INCI diethylamino hydroxybenzoyl hexyl benzoate); and
(ii) at least one water soluble UVA filter selected from [(3Z)-3-[[4-[(Z)-[7,7-dimethyl-2-oxo-1-(sulfomethyl)-3-bicyclo[2.2.1]heptanylidene]methyl]phenyl]methylidene]-7,7-dimethyl-2-oxo-1-bicyclo[2.2.1]heptanyl]methanesulfonic acid (INCI terephthalylidene dicamphor sulfonic acid);

wherein the composition does not comprise 2-hydroxy-4-methoxybenzophenone (INCI oxybenzone), ethylhexyl-2-cyano-3,3-diphenyl-acrylate (INCI octocrylene) and 2-ethylhexyl-(2E)-3-(4-methoxyphenyl)acrylate (INCI ethylhexyl methoxy cinnamate).

2.  The sunscreen or daily care composition according to claim 1, wherein the composition is free of parabens.

3.  The sunscreen or daily care composition according to claim 1 or 2, wherein the composition comprises at least one further UVA filter selected from the group consisting of triazine derivatives, dibenzoylmethane derivatives, benzotriazole derivatives and combinations thereof.

4.  The sunscreen or daily care composition according to claim 3, wherein the at least one further UVA filter is selected from the group consisting of 4-(tert.-butyl)-4'-methoxydibenzoylmethane (INCI butyl methoxydibenzoylmethane), 2,4-bis-{[4-(2-ethyl-hexyloxy)-2-hydroxy]-phenyl}-6-(4-methoxyphenyl)-1,3,5 triazine (INCI bis-ethylhexyloxyphenol methoxyphenyl triazine), 2-(2H-benzotriazol-2-yl)-4-methyl-6-[2-methyl-3-[1,3,3,3-tetramethyl-1-[(trimethylsilyl)oxy]-1-disiloxanyl]propyl]phenol (INCI drometrizole trisiloxane), 2-(2H-benzotriazol-2-yl)-6-(2-ethylhexyloxymethyl)-4-methyl-phenol and combinations thereof.

5.  The sunscreen or daily care composition according to any one of claims 1 to 4, wherein the composition further comprises at least one UVB filter selected from the group consisting of 4,4',4"-(1,3,5-triazin-2,4,6-triyltriimino)tris-benzoic acid-tris(2-ethylhexyl)ester (INCI ethylhexyl triazone), malonate derivatives such as dimethicone diethyl benzalmalonate, diethylhexyl butamido triazone, and combinations thereof.

6.  The sunscreen or daily care composition according to any one of claims 1 to 5, wherein the composition is free of phenoxyethanol.

7.  The sunscreen or daily care composition according to any one of claims 1 to 6, wherein the composition comprises hexyl 2-[4-(diethylamino)-2-hydroxybenzoyl]benzoate (INCI diethylamino hydroxybenzoyl hexyl benzoate) in an amount of from 1 % to 10 % by weight, based on the total weight of the composition.

8.  The sunscreen or daily care composition according to any one of claims 1 to 7, wherein the composition comprises the at least one water soluble UVA filter in an amount of from 0,1% to 10% by weight, preferably in an amount of from 0.3% to 5% by weight, more preferably in an amount of from 0.5% to 3% by weight, based on the total weight of the composition.

9.  The sunscreen or daily care composition according to any one of claims 1 to 8, wherein the composition is free of (RS)-2-ethylhexyl-2-hydroxybenzoate (INCI ethylhexyl salicylate) and 3,3,5-trimethyl-cyclohexyl salicylate (homo-salate).

Figure 1

Europäisches
Patentamt

European
Patent Office

Office européen
des brevets

## EUROPEAN SEARCH REPORT

Application Number

EP 22 16 5661

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 579 847 A1 (BEIERSDORF AG [DE]) 28 September 2005 (2005-09-28) * paragraph [0146]; example 6 * | 1-9 | INV. A61K8/41 A61K8/46 A61K8/49 A61Q17/04 |
| X | DE 10 2011 077037 A1 (BEIERSDORF AG [DE]) 13 December 2012 (2012-12-13) | 1,3-5, 7-9 | |
| Y | * example 7 * | 1-9 | |
| Y | EP 1 752 135 A1 (BEIERSDORF AG [DE]) 14 February 2007 (2007-02-14) * composition 14; pages 9-10 * * composition 5; pages 15-16 * * composition 1; pages 17-18 * * composition 5; pages 20-21 * | 1-9 | |
| Y | EP 3 354 253 A2 (BEIERSDORF AG [DE]) 1 August 2018 (2018-08-01) * claims 1-2 * * paragraph [0008] * * paragraph [0057]; example 1 * | 1-9 | TECHNICAL FIELDS SEARCHED (IPC) A61K A61Q |

The present search report has been drawn up for all claims

| Place of search | Date of completion of the search | Examiner |
|---|---|---|
| Munich | 12 July 2022 | Briand, Benoit |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 16 5661

This annex lists the patent family members relating to the patent documents cited in  the above-mentioned European search report.
The members are as contained in the European Patent Office EDP file on
The European Patent Office is in no way liable for these particulars which are merely given for the purpose of information.

12-07-2022

| Patent document cited in search report | | | Publication date | Patent family member(s) | | | Publication date |
|---|---|---|---|---|---|---|---|
| EP | 1579847 | A1 | 28-09-2005 | CH | 697392 | B1 | 15-09-2008 |
| | | | | DE | 102004020627 | A1 | 13-10-2005 |
| | | | | EP | 1579847 | A1 | 28-09-2005 |
| | | | | ES | 2656781 | T3 | 28-02-2018 |
| | | | | US | 2006008426 | A1 | 12-01-2006 |
| DE | 102011077037 | A1 | 13-12-2012 | BR | 112013031085 | A2 | 06-09-2016 |
| | | | | CN | 104053427 | A | 17-09-2014 |
| | | | | CN | 108635244 | A | 12-10-2018 |
| | | | | DE | 102011077037 | A1 | 13-12-2012 |
| | | | | EP | 2775997 | A2 | 17-09-2014 |
| | | | | ES | 2753583 | T3 | 13-04-2020 |
| | | | | US | 2014140940 | A1 | 22-05-2014 |
| | | | | WO | 2012167901 | A2 | 13-12-2012 |
| EP | 1752135 | A1 | 14-02-2007 | AT | 527988 | T | 15-10-2011 |
| | | | | DE | 102005037546 | A1 | 22-02-2007 |
| | | | | EP | 1752135 | A1 | 14-02-2007 |
| | | | | ES | 2375081 | T3 | 24-02-2012 |
| EP | 3354253 | A2 | 01-08-2018 | AU | 2018200455 | A1 | 09-08-2018 |
| | | | | BR | 102018001559 | A2 | 30-10-2018 |
| | | | | CN | 108403459 | A | 17-08-2018 |
| | | | | DE | 102017201235 | A1 | 26-07-2018 |
| | | | | EP | 3354253 | A2 | 01-08-2018 |
| | | | | ES | 2815452 | T3 | 30-03-2021 |

EPO FORM P0459

For more details about this annex : see Official Journal of the European Patent Office, No. 12/82

## REFERENCES CITED IN THE DESCRIPTION

**Patent documents cited in the description**

- EP 2837407 A2
- EP 3093008 A1
- EP 3093007 A1
- EP 3093006 A
- EP 3195853 A1

- EP 3351236 A1
- US 20030161793 A1
- DE 60022411 T2
- EP 3354253 A2